# EUROPEAN PATENT SPECIFICATION

(11) **EP 3 912 627 B1**
(45) Date of publication and mention of the grant of the patent: **07.09.2022**
(21) Application number: 20305525.6
(22) Date of filing: 20.05.2020
(51) Int. Cl.: A61K 31/4196, A61K 31/427, A61K 31/4706, A61K 31/513, A61K 31/706, A61K 45/06, A61P 31/14, A61K 31/685, A61K 38/21

(54) **METHODS FOR THE TREATMENT OF CORONAVIRUS INFECTIONS**
VERFAHREN ZUR BEHANDLUNG VON CORONAVIRUSINFEKTIONEN
PROCÉDÉS DE TRAITEMENT DES INFECTIONS PAR CORONAVIRUS

(43) Date of publication of application: 24.11.2021
(73) Proprietor: INSERM (Institut National de la Santé et de la Recherche Médicale), 75013 Paris (FR); Université Claude Bernard Lyon 1, 69100 Villeurbanne (FR); CENTRE NATIONAL DE LA RECHERCHE SCIENTIFIQUE -CNRS-, 75016 Paris (FR); Ecole Normale Supérieure de Lyon, 69007 Lyon (FR)
(72) Inventor: VIDALAIN, Pierre-Olivier, 69365 Lyon Cedex 07 (FR); LOTTEAU, Vincent, 69365 Lyon Cedex 07 (FR); JANIN, Yves Louis, 75724 Paris Cedex 15 (FR); NOUGAIREDE, Antoine, 13005 Marseille (FR); TOURET, Franck, 13005 Marseille (FR); DE LAMBALLERIE, Xavier, 13005 Marseille (FR); ANDRE, Patrice, 69365 Lyon Cedex 07 (FR)
(74) Representative: Inserm Transfert

(56) References cited:
- Rui Xiong ET AL: "Novel and potent inhibitors targeting DHODH, a rate-limiting enzyme in de novo pyrimidine biosynthesis, are broad-spectrum antiviral against RNA viruses including newly emerged coronavirus SARS-CoV-2", bioRxiv, 12 March 2020 (2020-03-12), XP055740305, DOI: 10.1101/2020.03.11.983056 Retrieved from the Internet: URL:http://biorxiv.org/lookup/doi/10.1101/ 2020.03.11.983056 [retrieved on 2020-10-15]
- CHRISTIAN SVEN ET AL: "The novel dihydroorotate dehydrogenase (DHODH) inhibitor BAY 2402234 triggers differentiation and is effective in the treatment of myeloid malignancies", BLOOD CANCER JOURNAL, NATURE PUBLISHING GROUP UK, LONDON, vol. 33, no. 10, 2 April 2019 (2019-04-02) , pages 2403-2415, XP036897088, ISSN: 0887-6924, DOI: 10.1038/S41375-019-0461-5 [retrieved on 2019-04-02]
- SINGH AWADHESH KUMAR ET AL: "Remdesivir in COVID-19: A critical review of pharmacology, pre-clinical and clinical studies", DIABETES & METABOLIC SYNDROME: CLINICAL RESEARCH & REVIEWS, ELSEVIER, AMSTERDAM, NL, vol. 14, no. 4, 12 May 2020 (2020-05-12), pages 641-648, XP086213966, ISSN: 1871-4021, DOI: 10.1016/J.DSX.2020.05.018 [retrieved on 2020-05-12]

### SEQUENCE LISTING

<110> INSERM
<120> METHODS FOR THE TREATMENT OF CORONAVIRUS INFECTIONS
<130> BIO20267 VIDALAIN / MC
<160> 3
<170> PatentIn version 3.3
<210> 1
   <211> 18
   <212> DNA
   <213> Artificial
<220>
   <223> Primer
<400> 1
   ggccgcaaat tgcacaat 18
<210> 2
   <211> 17
   <212> DNA
   <213> Artificial
<220>
   <223> primer
<400> 2
   ccaatgcgcg acattcc 17
<210> 3
   <211> 21
   <212> DNA
   <213> Artificial
<220>
   <223> probe
<400> 3
   cccccagcgc ttcagcgttc t 21

## Description

### FIELD:

The present disclosure is in the field of medicine, in particular virology.

### BACKGROUND:

*Coronaviridae* is a family of enveloped, positive-sense, single-stranded RNA viruses. The viral genome is 26-32 kilobases in length. The particles are typically decorated with large (~20 nm), club- or petal-shaped surface projections (the "peplomers" or "spikes"), which in electron micrographs of spherical particles create an image reminiscent of the solar corona. In late December 2019, a new betacoronavirus SARS-CoV-2 has emerged in Wuhan China [1-3]. The World Health Organization has named the severe pneumonia caused by this new coronavirus COVID-19 (for Corona Virus Disease 2019, WHO, 2020). Since its emergence, the SARS-CoV-2 has spread to 159 countries across the five continents causing, at the time of the writing, about 213,254 human infections with 81,238 cases in China (ECDC, March 19 2020). Europe has recently become the epicenter of COVID-19 epidemics with 82,869 confirmed cases; the majority of them being reported in Italy with 35,713 cases and 2978 deaths. In France, the number of confirmed cases is increasing with about 10,995 and 372 deaths on mid-march 2019 (Santé Publique France). To fight against the COVID-19 pandemic in a long term, in addition to the containment measures implemented in many countries, several projects have been launched around the world to understand the viral evolution and the pathophysiological consequences of the infection in order to identify therapeutic targets and to implement innovative therapies.

R. Xiong et al. discloses DHODH inhibitors and their antiviral effects against various RNA viruses, including SARS-CoV-2 (Rui Xiong et al. "Novel and potent inhibitors targeting DHODH, a rate-limiting enzyme in de novo pyrimidine biosynthesis, are broad-spectrum antiviral against RNA viruses including newly emerged coronavirus SARS-CoV-2", bioRxiv 2020.03.11.983056).

S. Christian et al. discloses BAY2402234 as a novel dihydroorotate dehydrogenase (DHODH) inhibitor effective in the treatment of myeloid malignancies, such as acute myeloid leukemia (S. Christian et al. "The novel dihydroorotate dehydrogenase (DHODH) inhibitor BAY 2402234 triggers differentiation and is effective in the treatment of myeloid malignancies". Leukemia 33, 2403-2415 (2019).

S. Awadhesh Kumar et al. discloses remdesivir as therapeutic agent in the treatment of COVID-19 (Singh, (Awadhesh Kumar et al. "Remdesivir in COVID-19: A critical review of pharmacology, pre-clinical and clinical studies." Diabetes & metabolic syndrome vol. 14,4 (2020): 641-648).

### SUMMARY:

As defined by the claims, the present invention relates to N-(2-Chloro-6-fluorophenyl)-4-[4-ethyl-3-(hydroxymethyl)-5-oxo-1,2,4-triazol-1-yl]-5-fluoro-2-[(2S)-1,1,1-trifluoropropan-2-yl]oxybenzamide for use in a method of treating a coronavirus infection in a subject in need thereof. The following detailed description, figures and example do not fall under the scope of the present invention and are present for understanding and illustration purposes only. In particular, the present invention is defined by the claims.

### DETAILED DESCRIPTION:

The present disclosure relates to N-(2-Chloro-6-fluorophenyl)-4-[4-ethyl-3-(hydroxymethyl)-5-oxo-1,2,4-triazol-1-yl]-5-fluoro-2-[(2S)-1,1,1-trifluoropropan-2-yl]oxybenzamide for use in a method of treating a coronavirus infection in a subject in need thereof.

As used herein, the term "coronavirus" has its general meaning in the art and refers to any member of members of the Coronaviridae family. Coronavirus is a virus whose genome is plus-stranded RNA of about 27 kb to about 33 kb in length depending on the particular virus. The virion RNA has a cap at the 5' end and a poly A tail at the 3' end. The length of the RNA makes coronaviruses the largest of the RNA virus genomes. In particular, coronavirus RNAs encode: (1) an RNA-dependent RNA polymerase; (2) N-protein; (3) three envelope glycoproteins; plus (4) three non-structural proteins. These coronaviruses infect a variety of mammals and birds. They cause respiratory infections (common), enteric infections (mostly in infants >12 mo.), and possibly neurological syndromes. Coronaviruses are transmitted by aerosols of respiratory secretions. Coronaviruses are exemplified by, but not limited to, human enteric coV (ATCC accession # VR-1475), human coV 229E (ATCC accession # VR-740), human coV OC43 (ATCC accession # VR-920), Middle East respiratory syndrome-related coronavirus (MERS-Cov) and SARS-coronavirus (Center for Disease Control), in particular SARS-Cov1 and SARS-Cov2.

According to the present disclosure, the compound of the present disclosure is particularly suitable for inhibiting the replication of the coronavirus as demonstrated in EXAMPLE.

In particular, the compound of the present disclosure is suitable for the treatment of Severe Acute Respiratory Syndrome (SARS). More particularly, the compound of the present disclosure is suitable for the treatment of COVID-19.

In particular, the subject can be human or any other animal (e.g., birds and mammals) susceptible to coronavirus infection (e.g. domestic animals such as cats and dogs; livestock and farm animals such as horses, cows, pigs, chickens, etc.). Typically said subject is a mammal including a non-primate (e.g., a camel, donkey, zebra, cow, pig, horse, goat, sheep, cat, dog, rat, and mouse) and a primate (e.g., a monkey, chimpanzee, and a human). In particular, the subject is a non-human animal. In particular, the subject is a farm animal or pet. In particular, the subject is a human. In particular, the subject is a human infant. In particular, the subject is a human child. In particular, the subject is a human adult. In particular, the subject is an elderly human. In particular, the subject is a premature human infant.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular interval, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

The compound of the present disclosure is also named BAY-2402234 and is described in WO2018077923 and Christian S, Merz C, Evans L, et al. The novel dihydroorotate dehydrogenase (DHODH) inhibitor BAY 2402234 triggers differentiation and is effective in the treatment of myeloid malignancies. Leukemia. 2019;33(10):2403 - 2415 as well as in Andreas Janzer, et al. Abstract DDT02-04: BAY 2402234: A novel, selective dihydroorotate dehydrogenase (DHODH) inhibitor for the treatment of myeloid malignancies. AACR Annual Meeting 2018; April 14-18, 2018; Chicago, IL*.* The compound has the formula of:

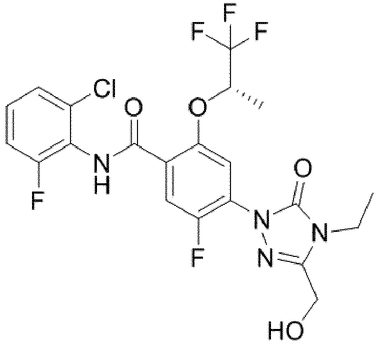

In particular, the compound of the present disclosure is administered to the subject in combination with at least one other therapeutic agent, preferably in combination with at least one other antiviral agent, more preferably in combination with at least one other antiviral agent selected from the group consisting of remdesivir, lopinavir, ritonavir, hydroxycholoroquine, and chloroquine.

In particular, the compound of the present disclosure is administered to the subject in combination with an interferon polypeptide.

As used herein, the term "interferon polypeptide" or "IFN polypeptide" is intended to include any polypeptide defined as such in the literature, comprising for example any types of IFNs (type I and type II) and in particular, IFN-alpha, IFN-beta, IFN-omega and IFN-gamma. The term interferon polypeptide, as used herein, is also intended to encompass salts, functional derivatives, variants, muteins, fused proteins, analogs and active fragments thereof. The polypeptide sequences for human interferon-alpha are deposited in database under accession numbers: AAA 52716, AAA 52724, and AAA 52713. The polypeptide sequences for human interferon-beta are deposited in database under accession numbers AAC41702, NP_002167, AAH 96152, AAH 96153, AAH 96150, AAH 96151, AAH 69314, and AAH 36040. The polypeptide sequences for human interferon-gamma are deposited in database under accession numbers AAB 59534, AAM 28885, CAA 44325, AAK 95388, CAA 00226, AAP 20100, AAP 20098, AAK 53058, and NP-000610.

In particular, the interferon is interferon-alpha (IFN-a). The term "IFN-a" encompasses derivatives of IFN-a that are derivatized (e.g., are chemically modified relative to the naturally occurring peptide) to alter certain properties such as serum half-life. As such, the term "IFN-a" includes IFN-a derivatized with polyethylene glycol ("PEGylated IFN-a"), and the like. PEGylated IFN-a, and methods for making same, is discussed in, e.g., U.S. Pat. Nos. 5,382,657; 5,951,974; and 5,981,709. PEGylated IFN-a encompasses conjugates of PEG and any of the above-described IFN-a molecules, including, but not limited to, PEG conjugated to interferon alpha-2a (Roferon, Hoffman La-Roche, Nutley, N.J.), interferon alpha-2b (Intron, Schering-Plough, Madison, N.J.), interferon alpha-2c (Berofor Alpha, Boehringer Ingelheim, Ingelheim, Germany); and consensus interferon as defined by determination of a consensus sequence of naturally occurring interferon alphas (Infergen^{®}, InterMune, Inc., Brisbane, CA.). Thus, in particular, the IFN-a has been modified with one or more polyethylene glycol moieties, i.e., pegylated. Two forms of pegylated-interferon, peginterferon alfa-2a (40 kD) (Pegasys, Hoffmann-La Roche) and peginterferon alfa-2b (12 kD) (Peglntron, Merck), are commercially available, which differ in terms of their pharmacokinetic, viral kinetic, tolerability profiles, and hence, dosing. In particular, Peginterferon alfa-2a (Pegasys) consists of interferon alfa-2a (~20 kD) covalently linked to a 40 kD branched polyethylene glycol (PEG). The PEG moiety is linked at a single site to the interferon alfa moiety via a stable amide bond to lysine. Peginterferon alfa-2a has an approximate molecular weight of 60,000 daltons. The biologic activity of peginterferon- alfa-2a derives from its interferon alfa-2a moiety which impacts both adaptive and innate immune responses against certain viruses. Compared with the native interferon alfa-2a, the peginterferon alfa-2a has sustained absorption, delayed clear. Peginterferon alfa-2a is used as a fixed weekly dose. Peginterferon alfa-2a has a relatively constant absorption after injection and is distributed mostly in the blood and organs.

By a "therapeutically effective amount" of the compound of the present disclosure is meant a sufficient amount of the compound to treat a coronavirus infection at a reasonable benefit/risk ratio applicable to any medical treatment. It will be understood, however, that the total daily usage of the compounds and compositions of the present disclosure will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination with the specific agonist employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

Typically, the compound of the present disclosure may be combined with pharmaceutically acceptable excipients, and optionally sustained-release matrices, such as biodegradable polymers, to form therapeutic compositions. "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present disclosure for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Galenic adaptations may be done for specific delivery in the small intestine or colon. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising the compound of the disclosure as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The compound of the disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifusoluble agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject. The compound of the disclosure may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered. In addition to compound formulated for parenteral administration, such as intravenous or intramuscular injection, other pharmaceutically acceptable forms include, e.g. tablets or other solids for oral administration; liposomal formulations; time release capsules; and any other form currently used.

The disclosure will be further illustrated by the following figures and examples.

### FIGURES:

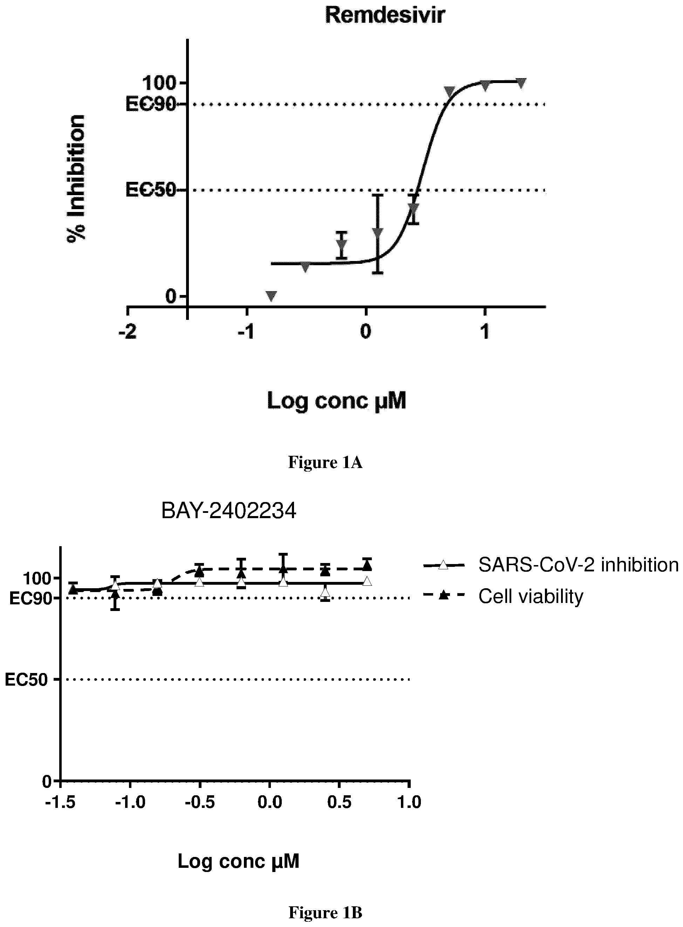

**Figure 1****. Effects of Remdesivir (A), and BAY-2402234 (B) on the replication of SARS-CoV-2 after two days of culture in Vero cells.** The effects on SARS-CoV-2 replication are expressed as percentages of inhibition compared to non-treated infected cultures. Dash lines indicate the EC50 and EC90 inhibition values. Cell viability is expressed as percentages of non-treated cells.

### EXAMPLE:

### Material & Methods

### Material:

SARS-CoV-2 strain BavPat1 was obtained from Pr Drosten through EVA GLOBAL (https://www.european-virus-archive.com/). Remdesivir (GS-5734), an adenosine analogue, was purchased from BLDpharm (China). BAY-2402234 was also synthetized.

### Methods:

### Determination of 50 and 90% effective concentrations (EC50 and EC90)

One day prior to infection, 5×10⁴ Vero E6 cells were seeded in 100µl assay medium (containing 2.5 % fetal calf serum [FCS]) in 96-well plates. The next day, eight 2 fold serial dilutions of compounds, in triplicates were added to the cells (25 µl/well, in 2.5 % FCS-containing medium). Four virus control wells (per virus) were supplemented with 25µL medium and four cell control wells were supplemented with 50 µl of medium. After 15 min, 25 µl of a virus mix diluted in medium was added to the wells at the correct MOI (0.002), determined so that the replication growth is still in the log growth curve for the readout at day 2. Plates were incubated for 2 days at 37°C after which 100 µl of the supernatant was collected for viral RNA purification.

**Table 1: Primers and probe used for RT-qPCR**

| **Primer/probe** | **Sequence (5' - 3')** | **Target** |
|---|---|---|
| NCoV_AN_F | GGCCGCAAATTGCACAAT (SEQ ID NO:1) | SARS-CoV-2 |
| NCoV_AN_R | CCAATGCGCGACATTCC (SEQ ID NO:2) | SARS-CoV-2 |
| NCoV_AN_P | FAM-CCCCCAGCGCTTCAGCGTTCT- BHQ1 (SEQ ID NO:3) | SARS-CoV-2 |

| | | |
|---|---|---|
| Reporter dye (FAM) and quencher (BHQ1) elements are indicated in bold and italics | | |

The 50% and 90% effective concentrations (EC50, EC90; the compound concentration that is required to inhibit viral RNA replication by 50% and 90 %) were determined using logarithmic interpolation.

### Results:

The effects of the reference antiviral compound remdesivir, which acts as a viral polymerase inhibitor, and BAY-2402234 are presented in **Figure 1****.** In this assay, the EC50 of remdesivir was 2.7 µM **(****Figure 1A**). In these conditions, BAY-2402234 has a much lower EC50 than remdesivir **(****Figure 1B****;** <40 nM). All together the data indicate that BAY-2402234 can repress the replication of SARS-CoV-2 in a significant manner and thus is suitable for the treatment of coronavirus infection.

### REFERENCES:

Throughout this application, various references describe the state of the art to which this disclosure pertains.

## Claims

1. N-(2-Chloro-6-fluorophenyl)-4-[4-ethyl-3-(hydroxymethyl)-5-oxo-1,2,4-triazol-1-yl]-5-fluoro-2-[(2S)-1,1,1-trifluoropropan-2-yl]oxybenzamide for use in a method of treating a coronavirus infection in a subject in need thereof.

2. N-(2-Chloro-6-fluorophenyl)-4-[4-ethyl-3-(hydroxymethyl)-5-oxo-1,2,4-triazol-1-yl]-5-fluoro-2-[(2S)-1,1,1-trifluoropropan-2-yl]oxybenzamide for use according to claim 1 wherein the compound is administered to the subject in combination with at least one other therapeutic agent, preferably in combination with at least one other antiviral agent, more preferably in combination with at least one other antiviral agent selected from the group consisting of remdesivir, lopinavir, ritonavir hydroxycholoroquine, and chloroquine.

3. N-(2-Chloro-6-fluorophenyl)-4-[4-ethyl-3-(hydroxymethyl)-5-oxo-1,2,4-triazol-1-yl]-5-fluoro-2-[(2S)-1,1,1-trifluoropropan-2-yl]oxybenzamide for use according to claim 1 wherein the compound is administered to the subject in combination with an interferon polypeptide.

## Patentansprüche

1. N-(2-Chloro-6-fluorophenyl)-4-[4-ethyl-3-(hydroxymethyl)-5-oxo-1,2,4-Triazol-1-yl]- 5-Fluor-2-[(2S)-1,1,1-Trifluoropropan-2-yl]oxybenzamid zur Verwendung in einem Verfahren zur Behandlung einer Coronavirus-Infektion in einem Subjekt, das diese benötigt.

2. N-(2-Chloro-6-fluorophenyl)-4-[4-ethyl-3-(hydroxymethyl)-5-oxo-1,2,4-Triazol-1-yl]- 5-Fluor-2-[(2S)-1,1,1-Trifluoropropan-2-yl]oxybenzamid zur Verwendung nach Anspruch 1, wobei die Zusammensetzung dem Subjekt in Kombination mit mindestens einem anderen Wirkstoff verabreicht wird, vorzugsweise in Kombination mit mindestens einem anderen antiviralen Mittel, insbesondere in Kombination mit mindestens einem anderen antiviralen Mittel, ausgewählt aus der Gruppe, bestehend aus Remdesivir, Lopinavir, Ritonavir Hydroxycholoroquin, und Chloroquin.

3. N-(2-Chloro-6-fluorophenyl)-4-[4-ethyl-3-(hydroxymethyl)-5-oxo-1,2,4-Triazol-1-yl]-5-Fluor-2-[(2S)-1,1,1-Trifluoropropan-2-yl]oxybenzamid zur Verwendung nach Anspruch 1, wobei die Zusammensetzung dem Subjekt in Kombination mit Interferonpolypeptid verabreicht wird .

## Revendications

1. N-(2-Chloro-6-fluorophényl)-4-[4-éthyl-3-(hydroxyméthyl)-5-oxo-1,2,4-triazol-1-yl]- 5-fluoro-2-[(2S)-1,1,1-trifluoropropan-2-yl]oxybenzamide pour utilisation dans une méthode de traitement d'une infection à coronavirus chez un sujet en ayant besoin.

2. N-(2-Chloro-6-fluorophényl)-4-[4-éthyl-3-(hydroxyméthyl)-5-oxo-1,2,4-triazol-1-yl]- 5-fluoro-2-[(2S)-1,1,1-trifluoropropan-2-yl]oxybenzamide pour utilisation selon la revendication 1 dans lequel le composé est administré au sujet en association avec au moins un autre agent thérapeutique, de préférence en association avec au moins un autre agent antiviral, plus préférentiellement en association avec au moins un autre agent antiviral sélectionné parmi le groupe consistant en le remdesivir, le lopinavir, le ritonavir, l'hydroxycholoroquine, et la chloroquine.

3. N-(2-Chloro-6-fluorophényl)-4-[4-éthyl-3-(hydroxyméthyl)-5-oxo-1,2,4-triazol-1-yl]- 5-fluoro-2-[(2S)-1,1,1-trifluoropropan-2-yl]oxybenzamide pour utilisation selon la revendication 1 dans lequel le composé est administré au sujet en association avec un polypeptide d'interféron.
